# FASCICULE DE BREVET EUROPEEN

(11) **EP 0 856 735 B1**
(45) Date de publication et mention de la délivrance du brevet: **25.04.2001**
(21) Numéro de dépôt: 98400041.4
(22) Date de dépôt: 12.01.1998
(51) Int. Cl.: G01N 33/80, G01N 1/30

(54) **Réactif de coloration pour la détermination de cellules sanguines**
Reagenz zur Färbung von Blutzellen
Colour staining reagent for blood cells

(30) Priorité: 31.01.1997 FR 9701090
(43) Date de publication de la demande: 05.08.1998
(73) Titulaire: ABX, 34184 Montpellier Cedex 4 (FR)
(72) Inventeur: Veriac, Sylvie, Les Jardins de l'Alhambra, 34070 Montpellier (FR)
(74) Mandataire: Bezault, Jean

(56) Documents cités:
- EP-A- 0 226 272
- EP-A- 0 430 719
- EP-A- 0 545 315
- US-A- 4 670 402
- DATABASE WPI Section Ch, Week 8622 Derwent Publications Ltd., London, GB; Class B04, AN 86-142078 XP002044489 & JP 61 079 163 A (TOA IYO DENSHI KK) , 22 avril 1986

## Description

L'invention concerne les analyses biologiques et notamment les analyses de sang.

Elle concerne plus particulièrement un réactif de coloration pour la détermination de cellules sanguines, en particulier de réticulocytes, du type comprenant un colorant propre à marquer les cellules après incubation.

On connaît déjà différents réactifs de coloration de ce genre qui permettent d'identifier et de caractériser des cellules sanguines grâce à un colorant qui permet de marquer ou colorer les cellules après incubation dans des conditions de durée et de température choisies.

De tels réactifs font généralement appel à un colorant fluorescent ou à un colorant non-fluorescent qui, après incubation, permet le comptage des cellules colorées. Ce comptage peut être réalisé avec une coloration manuelle au microscope, avec une coloration extérieure ou avec une coloration par un appareil automatisé.

Le comptage automatisé s'effectue généralement par une technique dite de cytométrie de flux qui s'avère plus fiable et plus rapide qu'un comptage manuel.

On connaît déjà différents colorants utilisés pour la détermination de cellules sanguines, en particulier de réticulocytes, et qui peuvent être utilisés pour un comptage manuel ou automatisé.

Parmi ces colorants fluorescents ou non-fluorescents, on peut citer notamment la pyronine Y, l'acridine orange, la thioflavine T, le thiazole orange, le nouveau bleu de méthylène ("new methylene blue"), le bleu crésyl brillant ("brilliant cresyl blue"), etc.

Des exemples de réactifs de coloration sont décrits notamment dans les publications de Brevets suivantes : CA 2 024 166, US 5 501 954, US 4 325 706, US 5 438 003, US 5 075 556, US 4 996 040, US 4 883 867, EP 0 545 314, EP 0 545 315, EP 0 430 719, EP 0 215 461, EP 0 226 272 et EP 0 114 462.

Dans le cas particulier de la détermination des réticulocytes, lesquels sont des précurseurs des érythrocytes ou globules rouges matures, le colorant sert à colorer ou marquer l'ARN résiduel contenu dans la cellule.

L'un des inconvénients des réactifs de coloration connus est qu'ils nécessitent un temps d'incubation élevé, ce qui rend difficile leur utilisation non seulement dans les techniques manuelles, mais surtout dans les techniques automatisées.

En particulier, le thiazole orange nécessite un temps d'incubation de l'ordre de 30 minutes à la température ambiante lorsqu'il est mis à réagir avec 5 microlitres de sang.

Ce temps d'incubation est beaucoup trop long pour permettre une automatisation complète du procédé.

On connaît par ailleurs, d'après US-A-4 670 402 et JP 61 079 163 A, des réactifs de coloration qui comprennent un détergent ou un agent tensio-actif. Toutefois, ces réactifs connus présentent aussi les inconvénients mentionnés ci-dessus.

L'invention a notamment pour but de surmonter les inconvénients précités.

C'est en particulier un but de l'invention de procurer un réactif de coloration pour la détermination de cellules sanguines, en particulier de réticulocytes, qui permet de marquer les cellules après un temps d'incubation beaucoup plus court que les temps d'incubation autorisés par les réactifs connus.

C'est aussi un but de l'invention de procurer un tel réactif de coloration qui permet d'abaisser le temps d'incubation de plusieurs minutes à plusieurs secondes.

L'invention propose à cet effet un réactif de coloration du type défini en introduction, lequel comprend en outre un additif propre à favoriser la pénétration du colorant dans la cellule, cet additif étant choisi parmi un composé ionophore et un mélange d'un tel composé ionophore avec un détergent.

Ainsi, le réactif de coloration de l'invention combine un colorant à un additif particulier qui favorise l'incorporation du colorant dans la cellule et, par conséquent, diminue notablement le temps d'incubation nécessaire à la réaction du colorant avec la cellule.

L'additif de l'invention comprend essentiellement un composé ionophore, c'est-à-dire un composé susceptible de favoriser la perméabilité de la membrane cellulaire et d'amplifier les échanges trans-membranaires.

Ces ionophores sont des molécules à caractère hydrophobe qui augmentent la perméabilité de la membrane cellulaire à certains ions avec une spécificité variable.

Ces ionophores sont soit des transporteurs mobiles, soit des molécules formant des canaux trans-membranaires. Ils masquent la charge de l'ion du colorant, facilitant sa pénétration dans la bi-couche lipidique de la membrane.

Le composé ionophore peut être mélangé avec un détergent, lequel favorise aussi la pénétration du colorant en favorisant la perméabilité de la membrane. Le détergent désorganise les structures protéiques de la membrane et favorise sa déstabilisation, ce qui contribue à une meilleure incorporation du colorant.

Le composé ionophore de l'invention peut être notamment un protonophore ou un antibiotique.

Des exemples non limitatifs de composés ionophores convenant à la mise en oeuvre de l'invention sont les suivants :
- Monensine, ou acide 2-[5-éthyltétrahydro-5-[tétrahydro-3-méthyl-5-[tétrahydro-6-hydroxy-6-(hydroxyméthyl)-3,5-diméthyl-2H-pyran-2-yl]-2-furyl]-2-furyl]-9-hydroxy-β-méthoxy-α,γ,2,8-tétraméthyl-1,6-dioxaspiro[4,5]décane-7-butyrique (polyéther antibiotique de formule brute : C₃₆H₆₂O₁₁);
- Nonactine, ou 2,5,11,14,20,23,29,32-octaméthyl-4,13,22,31,37,38,39,40-octaoxapentacyclo[32.2.1.1^{7,10}.1^{16,19}.1^{25,28}]-tétracontane-3,12,21,30-tétrone (macrotétrolide antibiotique de formule brute : C₄₀H₆₄O₁₂);
- 3,5-di-tert-butyl-4-hydroxy-benzylidènemalononitrile;
- Carbonylcyanide m-chlorophénylhydrazone;
- Carbonylcyanide p-trifluorométhoxyphénylhydrazone;
- Tétrachlorosalicylanilide;
- 4,5,6,7-tétrachloro-2-trifluorométhylbenzimidazole;
- Pentachlorophénol;
- 2,4-dinitrophénol;
- Valinomycine (cyclododécadepsi-peptide, antibiotique de formule : C₅₄H₉₀N₆O₁₈);
- Salinomycine (polyéther antibiotique de formule : C₄₂H₇₀O₁₁);
- Gramicidine(S) (polypeptide de formule : C₆₀H₉₂N₁₂O₁₀).

Le détergent pouvant être utilisé dans le réactif de coloration de l'invention est de préférence du type non-ionique ou de type zwittérionique.

A titre d'exemples non limitatifs de détergents convenant à la mise en oeuvre de l'invention, on peut mentionner :
- des propane sulfonates, en particulier le 3-[(3-cholamidopropyl)-diméthylammonio]-1-propane-sulfonate;
- des cholamides, en particulier le N,N-bis[3-D-gluconamidopropyl]-cholamide;
- des sulfobétaïnes;
- des alkyl glucosides et alkyl maltosides;
- des polyoxyéthylène éthers;
- des polyoxyéthylène sorbitans;
- des polyglycol éthers.

Le colorant utilisable dans le réactif de coloration de l'invention est un marqueur susceptible de révéler par coloration des composés intracellulaires tels que, par exemple, les acides nucléiques de la cellule, en particulier l'ARN.

Bien que l'on préfère utiliser un colorant fluorescent, et en particulier un colorant fluorescent pour les réticulocytes, il entre également dans le cadre de l'invention de faire appel à des colorants non fluorescents.

Lorsqu'il s'agit d'un colorant fluorescent, le colorant est avantageusement excitable à une longueur d'onde comprise entre 0,1 nm et 1 mm.

De préférence, il s'agit d'un colorant excitable à la lumière bleue, en particulier à une longueur d'onde de 488 nm.

A titre d'exemples non limitatifs de colorants utilisables dans la mise en oeuvre de l'invention, on peut mentionner :
- le 3,3'-diméthyloxacarbocyanine iodure (ou 3-méthyl-2-[3-(3-méthyl-2(3H)-benzoxazolylidène)-1-propényl]benzoxazolium iodure);
- le thiazole orange ou 1-méthyl-4-[(3-méthyl-2-(3H)-benzothiazolylidène)méthyl]quinolinium p-tosylate;
- le quinolinium, 4-[(3-méthyl-2-(3H)-benzothiazolylidène)méthyl]-1-[3-(triméthylammonio)propyl],diiodure (commercialisé sous la dénomination TO-PRO 1, Marque déposée par Molecular Probes);
- des styryls, en particulier le styryl 7;
- le nouveau bleu de méthylène;
- le bleu de crésyl brillant.

Lorsque ce colorant connu est combiné dans des proportions choisies à un composé ionophore seul ou mélangé avec un détergent, il franchit rapidement la membrane de la cellule, ce qui permet de diminuer notablement le temps d'incubation et de l'amener généralement à une valeur de quelques dizaines de secondes.

Ainsi, dans le cas du thiazole orange, le temps d'incubation peut être amené à une valeur de l'ordre de 25 secondes, au lieu de 30 minutes à la température ambiante, lorsque le colorant est utilisé seul.

Le réactif de coloration de l'invention peut comporter d'autres composés ou substances que le colorant et l'additif mentionnés précédemment.

Ainsi, le réactif de coloration peut comprendre en outre un solvant organique, en particulier un alcool tel que le méthanol, ce qui favorise non seulement la mise en solution du colorant, mais aussi la solubilisation des lipides membranaires de la cellule.

En variante ou en complément, le réactif de coloration peut comporter en outre un sel, par exemple à base de sodium, de potassium.

Il a été constaté en effet qu'une concentration en sels induit une force dite "ionique" qui, si elle est suffisamment élevée, peut déstabiliser les membranes cellulaires en modifiant les liaisons des constituants membranaires. De plus, le sel permet d'agir sur le volume des cellules.

D'autres composés pouvant faire partie du réactif de l'invention comprennent un agent chélateur, en particulier l'EDTA, un conservateur et un système tampon pour maintenir le pH entre 5 et 11.

Le réactif-type de l'invention est une solution de coloration ou de marquage qui contient :
- un colorant à une concentration comprise entre 0,1 µM et 0,5 M
- au moins un additif choisi parmi un composé ionophore à une concentration de 0-1 M et un mélange d'un tel composé ionophore avec un détergent à concentration de 0-20%
- un ou plusieurs des composés énumérés ci-dessous :

| Composés | Concentrations |
|---|---|
| Sels (NaCl/KCl) | 0-1 M |
| Agent chélateur (EDTA) | 0-100 mM |
| Solvant | 0-15% |
| Conservateur | 0-1% |

- un système tampon organique ou inorganique maintenant le pH entre 5 et 11.

Un exemple de réactif de coloration selon l'invention est le suivant :

| Composés | Concentrations |
|---|---|
| thiazole orange | 2 µM |
| valinomycine | 1 µM |
| polyglycoléther | 0,0003% |
| NaCl | 155 mM |
| EDTA | 2 mM |
| méthanol | 1,5% |

Le système tampon utilisé est un tampon phosphate ajustant le pH de la solution de coloration à valeur neutre.

L'invention sera maintenant expliquée en référence aux dessins annexés qui représente des images obtenues sur un cytomètre de flux et qui comparent à chaque fois l'image obtenue avec un colorant de référence et l'image obtenue avec un réactif de coloration selon l'invention.

Ces images représentent la fluorescence (axe des X) et la diffraction (axe des Y). On retrouve sur chacune de ces figures, de la gauche vers la droite, respectivement, trois populations : la population des globules rouges (apparaissant sous la forme d'un nuage), la population des réticulocytes (partie encadrée) et la population des leucocytes (nuage dispersé).

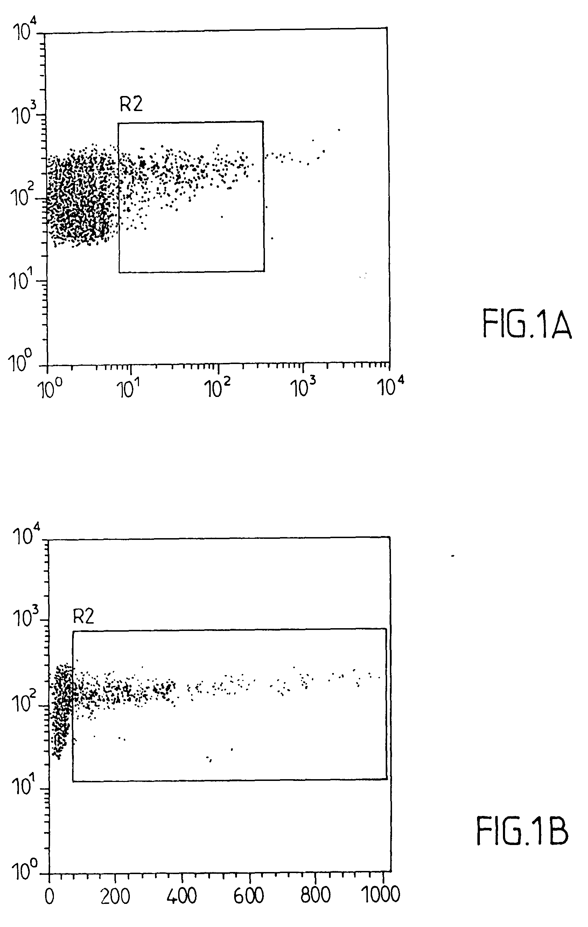

Les figures 1A et 1B représentent les images obtenues sur un cytomètre de flux FACSCAN (Marque déposée de Becton Dickinson) en utilisant le thiazole orange comme colorant.

La figure lA représente l'image obtenue avec le colorant de référence. Le temps d'incubation requis est de 30 minutes à la température ambiante. Le taux de réticulocytes est de 4,55%.

La figure 1B montre l'image obtenue avec un réactif de coloration selon l'invention, qui comporte du thiazole orange en présence de ionophore. Le temps d'incubation nécessaire n'est que de 25 secondes à 35°C, le taux de réticulocytes étant de 4,89%.

Les figures 2A et 2B sont des images obtenues avec un cytomètre de flux FACSCAN en utilisant un réactif de coloration selon l'invention, qui comporte du 3,3'-diméthylcarbocyanine iodure en tant que colorant, en présence d'un ionophore et d'un détergent.

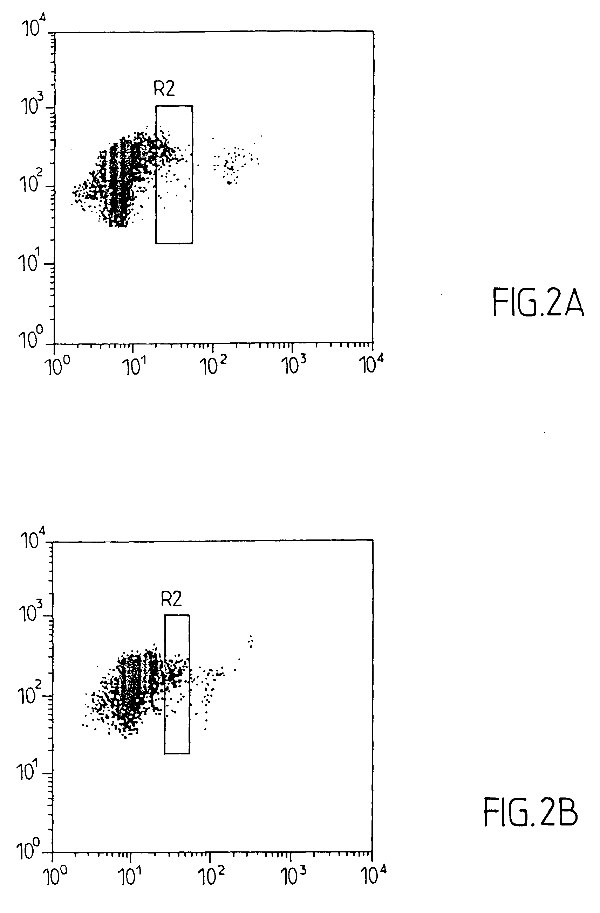

Les deux colorations sont réalisées à 35°C. On obtient un résultat équivalent à 5 minutes pour le colorant de référence (figure 2A) et à 30 secondes pour le réactif selon l'invention (figure 2B).

Les taux de réticulocytes des images des figures 2A et 2B sont respectivement de 1,5% et de 1,3%.

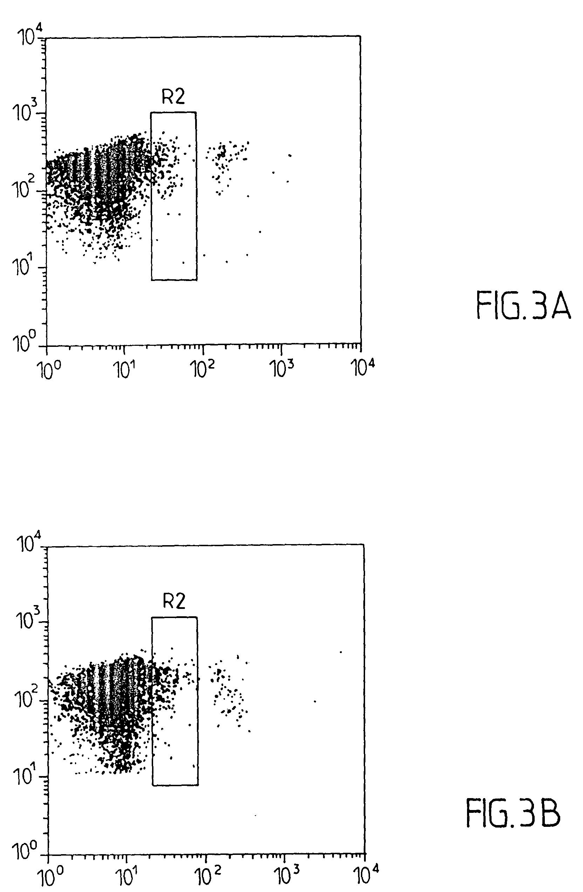

Les figures 3A et 3B sont des images obtenues sur un cytomètre de flux FACSCAN avec le colorant TO-PRO 1 (Marque déposée de Molecular Probes), sous irradiation à la lumière bleue.

Dans le cas de la figure 3A (colorant de référence), le temps d'incubation est de 60 minutes et le taux de réticulocytes de 2,22.

Dans le cas de la figure 3B (colorant en présence de ionophore), le temps d'incubation est de 5 minutes et le taux de réticulocytes de 2,14%.

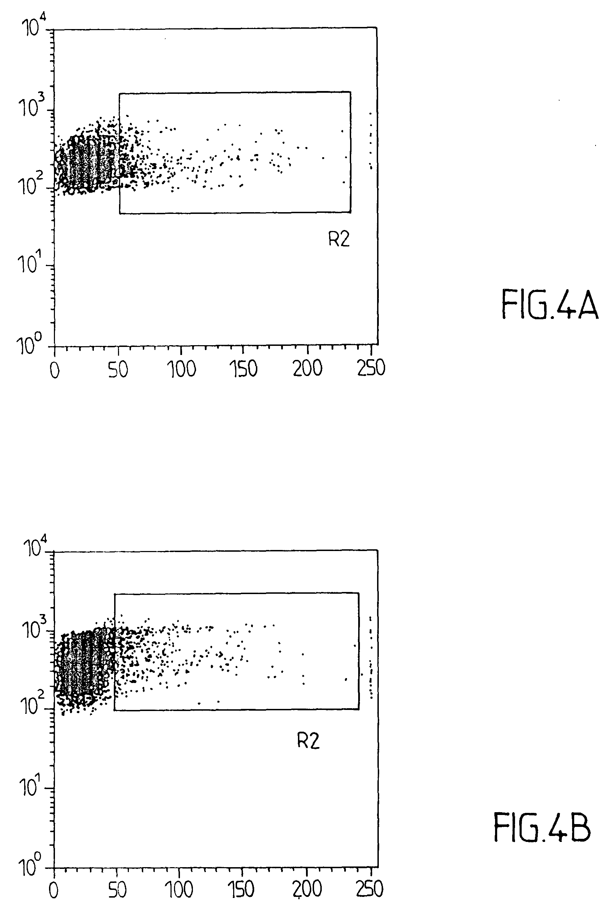

Les figures 4A et 4B représentent des images obtenues sur un cytomètre de flux FACSTAR (Marque déposée de Becton Dickinson) avec un colorant du type TO-PRO 3 (Marque déposée de Molecular Probes), sous irradiation à la lumière rouge.

Dans le cas de la figure 4A (colorant de référence), le temps d'incubation est de 30 minutes et le taux de réticulocytes de 4,1%.

Dans le cas de la figure 4B (colorant en présence de ionophore), le temps d'incubation est de 2 minutes et le taux de réticulocytes de 4,9%.

Les images précédentes montrent que l'utilisation d'un réactif de coloration selon l'invention permet de diminuer significativement le temps d'incubation par rapport à l'utilisation d'un colorant de référence.

## Revendications

1. Réactif de coloration pour la détermination de cellules sanguines, en particulier de réticulocytes, du type comprenant un colorant propre à marquer les cellules après incubation,
caractérisé en ce qu'il comprend en outre un additif propre à favoriser la pénétration du colorant dans les cellules, cet additif étant choisi parmi un composé ionophore, et un mélange d'un tel composé ionophore avec un détergent.

2. Réactif de coloration selon la revendication 1, caractérisé en ce que l'additif comprend un composé ionophore seul.

3. Réactif selon la revendication 1, caractérisé en ce que l'additif comprend un mélange d'un composé ionophore et d'un détergent.

4. Réactif selon l'une des revendications 1 à 3, caractérisé en ce que le composé ionophore est un protonophore ou un antibiotique choisi parmi :
- Monensine ou acide 2-[5-éthyltétrahydro-5-[tétrahydro-3-méthyl-5-[tétrahydro-6-hydroxy-6-(hydroxyméthyl)-3,5-diméthyl-2H-pyran-2-yl]-2-furyl]-2-furyl]-9-hydroxy-β-méthoxy-α-,γ,2,8-tétraméthyl-1,6-dioxaspiro[4,5]décane-7-butyrique;
- Nonactine ou 2,5,11,14,20,23,29,32-octaméthyl-4,13,22,31,37,38,39,40-octaoxapentacyclo[32.2.1.1^{7,10}.1^{16,19}.1^{25,28}]-tétracontane-3,12,21,30-tétrone;
- 3,5-di-tert-butyl-4-hydroxy-benzylidènemalononitrile;
- Carbonylcyanide m-chlorophénylhydrazone;
- Carbonylcyanide p-trifluorométhoxyphénylhydrazone;
- Tétrachlorosalicylanilide;
- 4,5,6,7-tétrachloro-2-trifluorométhylbenzimidazole;
- Pentachlorophénol;
- 2,4-dinitrophénol;
- Valinomycine;
- Salinomycine;
- Gramicidine(S).

5. Réactif de coloration selon l'une des revendications 1 et 3, caractérisé en ce que le détergent est du type non-ionique ou zwittérionique et choisi parmi :
- des propane sulfonates, en particulier le 3-[(3-cholamidopropyl)-diméthylammonio]-1-propane-sulfonate;
- des cholamides, en particulier le N,N-bis[3-D-gluconamidopropyl]-cholamide;
- des sulfobétaïnes;
- des alkyl glucosides et alkyl maltosides;
- des polyoxyéthylène éthers;
- des polyoxyéthylène sorbitans;
- des polyglycol éthers.

6. Réactif de coloration selon l'une des revendications 1 à 5, caractérisé en ce que le colorant est du type fluorescent.

7. Réactif de coloration selon l'une des revendications 1 à 6, caractérisé en ce que le colorant est excitable à une longueur d'onde comprise entre 0,1 nm et 1 mm.

8. Réactif de coloration selon l'une des revendications 1 à 7, caractérisé en ce que le colorant est excitable à la lumière bleue, en particulier à une longueur d'onde de 488 nm.

9. Réactif de coloration selon l'une des revendications 1 à 8, caractérisé en ce que le colorant est choisi parmi les suivants :
- le 3,3'-diméthyloxacarbocyanine iodure (ou 3-méthyl-2-[3-(3-méthyl-2(3H)-benzoxazolylidène)-1-propényl]benzoxazolium iodure);
- le thiazole orange ou 1-méthyl-4-[(3-méthyl-2-(3H)-benzothiazolylidène)méthyl]quinolinium p-tosylate;
- le quinolinium, 4-[(3-méthyl-2-(3H)-benzothiazolylidène)méthyl]-1-[3-(triméthylammonio)propyl], diiodure;
- des styryls, en particulier le styryl 7;
- le nouveau bleu de méthylène;
- le bleu de crésyl brillant.

10. Réactif de coloration selon l'une des revendications 1 à 9, caractérisé en ce qu'il comprend en outre au moins un composé choisi parmi les suivants :
- un sel, en particulier de sodium ou de potassium,
- un agent chélateur, en particulier l'EDTA,
- un solvant, en particulier un alcool,
- un conservateur, et
- un système tampon maintenant le pH entre 5 et 11.

11. Réactif de coloration selon l'une des revendications 1 à 10, caractérisé par la composition suivante :
- un colorant à une concentration comprise entre 0,1 µM et 0,5 M
- au moins un additif choisi parmi un composé ionophore à une concentration de 0-1 M et un mélange d'un tel composé ionophore avec un détergent à concentration de 0-20%
- un ou plusieurs des composés énumérés ci-dessous :
| Composés | Concentrations |
|---|---|
| Sels (NaCl/KCl) | 0-1 M |
| Agent chélateur (EDTA) | 0-100 mM |
| Solvant | 0-15% |
| Conservateur | 0-1% |
- un système tampon organique ou inorganique maintenant le pH entre 5 et 11.

## Patentansprüche

1. Färbereagens für die Bestimmung von Blutzellen, insbesondere von Retikulozyten, wobei das Färbereagens ein Färbemittel aufweist, das geeignet ist, die Zellen nach einer Inkubation zu markieren,
dadurch gekennzeichnet,
daß es des weiteren einen Zusatz enthält, der geeignet ist, das Eindringen des Färbemittels in die Zellen zu fördern, wobei dieser Zusatz aus einer ionophoren Zusammensetzung und einer Mischung einer solchen ionophoren Zusammensetzung mit einem Detergens ausgewählt ist.

2. Färbereagens nach Anspruch 1,
dadurch gekennzeichnet,
daß der Zusatz nur eine ionophore Zusammensetzung aufweist.

3. Reagens nach Anspruch 1,
dadurch gekennzeichnet,
daß der Zusatz eine Mischung aus einer ionophoren Zusammensetzung und aus einem Detergens aufweist.

4. Reagens nach einem der Ansprüche 1 bis 3,
dadurch gekennzeichnet,
daß die ionophore Zusammensetzung ein Protonophor oder ein Antibiotikum ist, das ausgewählt ist aus der folgenden Gruppe:
- Monensin oder 2-[5-ethyltetrahydro-5-[tetrahydro-3-methyl-5-[tetrahydro-6-hydroxy-6-(hydroxymethyl)-3,5-dimethyl-2H-pyran-2-yl]-2-furyl]-2-furyl]-9-hydroxy-β-methoxy-α,γ,2,8-tetramethyl-1,6-dioxaspiro [4,5]decan-7-Buttersäure;
- Nonactin oder 2,5,11,14,20,23,29,32-octamethyl-4,13,22,31,37,38,39,40-octaoxapentacyclo [32.2.1.1^{7,10}.1^{16,19}.1^{25,28}]-tetracontan-3,12,21,30-tetron;
- 3,5-Di-tert-butyl-4-hydroxy-benzylidenmalononitril;
- Carbonylcyanid m-chlorphenylhydrazon;
- Carbonylcyanid p-trifluormethoxyphenylhydrazon;
- Tetrachlorsalicylanilid;
- 4,5,6,7-Tetrachlor-2-trifluormethylbenzimidazol;
- Pentachlorphenol;
- 2,4-Dinitrophenol;
- Valinomycin;
- Salinomycin;
- Gramicidin(S).

5. Färbereagens nach einem der Ansprüche 1 und 3,
dadurch gekennzeichnet,
daß das Detergens nichtionisch oder zwitterionisch ist und ausgewählt ist aus der folgenden Gruppe:
- Propansulfonaten, insbesondere 3-[(3-Cholamidopropyl)-dimethylammonium]-1-propan-sulfonat;
- Cholamiden, insbesondere N,N-bis[3-D-gluconamidopropyl]-cholamid;
- Sulfobetainen;
- Alkylglucosiden und Alkylmaltosiden;
- Polyoxyethylenethern;
- Polyoxyethylensorbitanen;
- Polyglycolethern.

6. Färbereagens nach einem der Ansprüche 1 bis 5,
dadurch gekennzeichnet,
daß das Färbemittel von einem fluoreszierenden Typ ist.

7. Färbereagens nach einem der Ansprüche 1 bis 6,
dadurch gekennzeichnet,
daß das Färbemittel bei einer Wellenlänge im Bereich von 0,1 nm bis 1 mm anregbar ist.

8. Färbereagens nach einem der Ansprüche 1 bis 7,
dadurch gekennzeichnet,
daß das Färbemittel mit blauem Licht, insbesondere bei einer Wellenlänge von 488 nm anregbar ist.

9. Färbereagens nach einem der Ansprüche 1 bis 8,
dadurch gekennzeichnet,
daß das Färbemittel aus den folgenden Substanzen ausgewählt ist:
- 3,3'-Dimethyloxacarbocyaninjodit (oder 3-methyl-2-[3-(3-methyl-2(3H)-benzoxazolyliden)-1-propenyl]benzoxazoliumjodit);
- Thiazolorange oder 1-Methyl-4-[(3-methyl-2-(3H)-benzothiazolyliden)methyl]chinolin p-tosylat;
- Chinolin, 4-[(3-methyl-2-(3H)-benzothiazolyliden) methyl]-1-[3- (trimethylammonium)propyl], dijodit;
- Styryl, insbesondere Styryl 7;
- neuem Methylenblau;
- Cresylbrilliantblau.

10. Färbereagens nach einem der Ansprüche 1 bis 9,
dadurch gekennzeichnet,
daß es weiterhin wenigstens eine Zusammensetzung enthält, die aus den folgenden ausgewählt ist:
- einem Salz, insbesondere von Natrium oder von Kalium,
- einem Chelatbildner, insbesondere EDTA,
- einem Lösungsmittel, insbesondere einem Alkohol,
- einem Konservierungsmittel, und
- einem Puffersystem, das den pH-Wert im Bereich zwischen 5 und 11 hält.

11. Färbereagens nach einem der Ansprüche 1 bis 10, gekennzeichnet durch die folgende Zusammensetzung:
- ein Färbemittel in einer Konzentration, die im Bereich zwischen 0,1 *µ*M und 0,5 M liegt,
- wenigstens einen Zusatz, der aus einer ionophoren Zusammensetzung ausgewählt ist in einer Konzentration im Bereich von 0 M bis 1 M und einer Mischung einer solchen ionophoren Zusammensetzung mit einem Detergens in einer Konzentration im Bereich von 0 % bis 20 %,
- eine oder mehrere der nachstehend aufgezählten Zusammensetzungen:
| Zusammensetzungen | Konzentrationen |
|---|---|
| Salze (NaCl/KCl) | 0 - 1 M |
| Chelatbildner (EDTA) | 0 - 100 mM |
| Lösungsmittel | 0 - 15 % |
| Konservierungsmittel | 0 - 1 % |
- ein organisches oder anorganisches Puffersystem, das den pH-Wert im Bereich zwischen 5 und 11 hält.

## Claims

1. Staining reagent for the determination of blood cells, in particular reticulocytes, of the type comprising a dye which is capable of labelling the cells after incubation,
characterized in that it also comprises an additive which is capable of promoting the penetration of the dye into the cells, this additive being chosen from an ionophoric compound, and a mixture of such an ionophoric compound with a detergent.

2. Staining reagent according to Claim 1,
characterized in that the additive comprises an ionophoric compound alone.

3. Reagent according to Claim 1, characterized in that the additive comprises a mixture of an ionophoric compound and a detergent.

4. Reagent according to one of Claims 1 to 3,
characterized in that the ionophoric compound is a protonophor or an antibiotic chosen from:
- monensin or 2- [5-ethyltetrahydro-5-[tetrahydro-3-methyl-5-[tetrahydro-6-hydroxy-6-(hydroxymethyl)-3,5-dimethyl-2H-pyran-2-yl]-2-furyl]-9-hydroxy-*β* -methoxy-α,γ,2,8-tetramethyl-1,6-dioxaspiro[4,5]decane-7-butyric acid;
- nonactin or 2,5,11,14,20,23,29,32-octamethyl-4,13,22,31,37,38,39,40-octaoxapentacyclo-[32.2.1.1^{7,10}.1^{16,19}.1^{25,28}] tetracontane-3,12,21,30-tetrone;
- 3,5-di-tert-butyl-4-hydroxybenzylidenemalononitrile;
- carbonylcyanide m-chlorophenylhydrazone;
- carbonylcyanide p-trifluoromethoxyphenylhydrazone;
- tetrachlorosalicylanilide;
- 4,5,6,7-tetrachloro-2-trifluoromethylbenzimidazole;
- pentachlorophenol;
- 2,4-dinitrophenol;
- valinomycin;
- salinomycin;
- (S)-gramicidin.

5. Staining reagent according to either of Claims 1 and 3, characterized in that the detergent is of the nonionic or zwitterionic type and is chosen from:
- propane sulphonates, in particular 3-[(3-cholamidopropyl)dimethylammonio]-1-propane sulphonate;
- cholamides, in particular N,N-bis[3-D-gluconamidopropyl]cholamide;
- sulphobetaines;
- alkyl glucosides and alkyl maltosides;
- polyoxyethylene ethers;
- polyoxyethylene sorbitans;
- polyglycol ethers.

6. Staining reagent according to one of Claims 1 to 5, characterized in that the dye is of the fluorescent type.

7. Staining reagent according to one of Claims 1 to 6, characterized in that the dye is excitable at a wavelength of between 0.1 nm and 1 mm.

8. Staining reagent according to one of Claims 1 to 7, characterized in that the dye is excitable with blue light, in particular at a wavelength of 488 nm.

9. Staining reagent according to one of Claims 1 to 8, characterized in that the dye is chosen from the following:
- 3,3-dimethyloxacarbocyanine iodide (or 3-methyl-2-[3-(3-methyl-2-(3H)-benzoxazolylidene)-1-propenyl]benzoxazolium iodide);
- thiazole orange or 1-methyl-4-[(3-methyl-2-(3H)-benzothiazolylidene)methyl]quinolinium p-tosylate;
- quinolinium, 4-[(3-methyl-2-(3H)-benzothiazolylidene)methyl]-1-[3-(trimethylammonio)propyl] diiodide;
- styryls, in particular styryl 7;
- new methylene blue;
- brilliant cresyl blue.

10. Staining reagent according to one of Claims 1 to 9, characterized in that it also comprises at least one compound chosen from the following:
- a salt, in particular a sodium or potassium salt,
- a chelating agent, in particular EDTA,
- a solvent, in particular an alcohol,
- a preserving agent, and
- a buffer system for maintaining the pH between 5 and 11.

11. Staining reagent according to one of Claims 1 to 10, characterized by the following composition:
- a dye at a concentration of between 0.1 µM and 0.5 M,
- at least one additive chosen from an ionophoric compound at a concentration of 0-1 M and a mixture of such an ionophoric compound with a detergent at a concentration of 0-20%,
- one or more of the compounds listed below:
| Compounds | Concentrations |
|---|---|
| Salts (NaCl/KCl) | 0-1 M |
| Chelating agent (EDTA) | 0-100 mM |
| Solvent | 0-15% |
| Preserving agent | 0-1% |
- an organic or inorganic buffer system for maintaining the pH between 5 and 11.
